(19) Europäisches Patentamt
European Patent Office
Office européen des brevets

(11) Veröffentlichungsnummer: **0 486 798 A1**

(12) **EUROPÄISCHE PATENTANMELDUNG**

(21) Anmeldenummer: **91117032.2**

(22) Anmeldetag: **07.10.91**

(51) Int. Cl.5: **A01N 43/82**, C07D 271/10, C07D 285/12

(30) Priorität: **20.10.90 DE 4033412**

(43) Veröffentlichungstag der Anmeldung: **27.05.92 Patentblatt 92/22**

(84) Benannte Vertragsstaaten: **BE CH DE DK ES FR GB GR IT LI NL**

(71) Anmelder: **BAYER AG**

**W-5090 Leverkusen 1 Bayerwerk(DE)**

(72) Erfinder: **Kleefeld, Gerd, Dr.**
**Franziskusstrasse 27**
**W-4040 Neuss-Uedesheim(DE)**
Erfinder: **Diehr, Hans-Joachim, Dr.**
**Höhe 35**
**W-5600 Wuppertal 11(DE)**
Erfinder: **Haas, Wilhelm, Dr.**
**Nordstrasse 1**
**W-5014 Kerpen 3(DE)**
Erfinder: **Dehne, Heinz-Wilhelm, Dr.**
**Krischer Strasse 81**
**W-4019 Monheim(DE)**
Erfinder: **Brandes, Wilhelm, Dr.**
**Eichendorffstrasse 3**
**W-5653 Leichlingen 1(DE)**

(54) **Fungizide Mittel auf Basis von heterocyclisch substituierten Sulfonen.**

(57) Verwendung von Verbindungen der Formel (I),

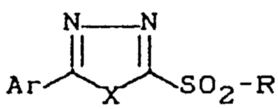

(I)

in welcher
Ar für einfach bis dreifach, gleich oder verschieden substituiertes Phenyl steht,
R für geradkettiges oder verzweigtes Alkyl mit 1 bis 4 Kohlenstoffatomen steht und
X für Sauerstoff oder Schwefel steht,
zur Bekämpfung von phytopathogenen Pilzen in der Landwirtschaft.

Die Verbindungen der Formel (I) sind bekannt und können nach Analogieverfahren hergestellt werden, z.B. indem man die entsprechenden Thioverbindungen oxydiert, z.B. mit Kaliumpermanganat.

EP 0 486 798 A1

Die Erfindung betrifft die Verwendung von heterocyclisch substituierten Sulfonen als Fungizide gegen phytopathogene Krankheitserreger in der Landwirtschaft.

Es ist bekannt, daß bestimmte heterocyclisch substituierte Sulfone wie beispielsweise die Verbindung 2-[(4-Methyl-3-nitrophenyl)-sulfonyl]-5-trifluormethyl-1,3,4-thiadiazol eine fungizide Wirksamkeit gegenüber phytopathogenen Krankheitserregern in der Landwirtschaft besitzen (vergleiche z. B. DE 25 33 604).

Die Wirksamkeit dieser vorbekannten Verbindungen ist jedoch insbesondere bei niedrigen Aufwandmengen und Konzentrationen nicht in allen Anwendungsgebieten völlig zufriedenstellend.

Weiterhin ist bekannt, daß bestimmte heterocyclisch substituierte Sulfone, wie beispielsweise die Verbindung 2-Methylsulfonyl-5-(3-nitrophenyl)-1,3,4-oxadiazol oder die Verbindung 2-(2-Chlorphenyl)-5-methylsulfonyl-1,3,4-oxadiazol bei in vitro-Versuchen eine gewisse fungistatische Wirkung gegen Aspergillus niger und flavus besitzen (vergleiche z. B. Agric. Biol. Chem. 40, 17-21 [1976] bzw. CA 84: 121736n).

Es wurde nun gefunden, daß heterocyclisch substituierte Sulfone der allgemeinen Formel (I),

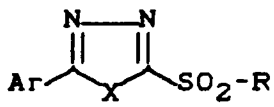

(I)

in welcher

Ar für einfach bis dreifach, gleich oder verschieden substituiertes Phenyl steht, wobei als Substituenten infrage kommen: Halogen, Cyano, Nitro, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen, jeweils geradkettiges oder verzweigtes Alkoxycarbonyl oder Alkoximinoalkyl mit jeweils 1 bis 4 Kohlenstoffatomen in den einzelnen Alkylteilen oder gegebenenfalls einfach bis fünffach, gleich oder verschieden durch Halogen und/oder geradkettiges oder verzweigtes Alkyl mit 1 bis 4 Kohlenstoffatomen substituiertes Phenyl,

R für geradkettiges oder verzweigtes Alkyl mit 1 bis 4 Kohlenstoffatomen steht und

X für Sauerstoff oder Schwefel steht, ausgenommen die Verbindungen 5-(2-Chlorphenyl)- und 5-(3-Nitrophenyl)-2-methylsulfonyl-1,3,4-oxadiazol,

eine besonders gute Wirkung gegen landwirtschaftlich relevante phytopathogene Pilzarten besitzen.

Überraschenderweise zeigen die erfindungsgemäß verwendbaren heterocyclisch substituierten Sulfone der allgemeinen Formel (I) eine erheblich bessere fungizide Wirksamkeit gegenüber phytopathogenen Pilzen in der Landwirtschaft als die aus dem Stand der Technik bekannten heterocyclisch substituierten Sulfone.

Die erfindungsgemäß verwendbaren heterocyclisch substituierten Sulfone sind durch die Formel (I) allgemein definiert. Bevorzugt verwendbar sind Verbindungen der Formel (I), bei welchen

Ar für ein- bis dreifach, gleich oder verschieden substituiertes Phenyl steht, wobei als Substituenten infrage kommen: Fluor, Chlor, Brom, Cyano, Nitro, Methyl, Ethyl, n- oder i-Propyl, n-, i-, s oder t-Butyl, Methoxy, Ethoxy, n- oder i-Propoxy, Methylthio, Difluormethyl, Trifluormethyl, Trifluormethoxy, Trifluormethylthio, Methoxycarbonyl, Ethoxycarbonyl, Methoximinomethyl, Ethoximinomethyl, Methoximinoethyl, Ethoximinoethyl oder gegebenenfalls ein- bis dreifach, gleich oder verschieden durch Fluor, Chlor, Brom, Methyl und/ oder Ethyl substituiertes Phenyl,

R für Methyl oder Ethyl steht und

X für Sauerstoff steht, oder

Ar für ein- bis dreifach, gleich oder verschieden substituiertes Phenyl steht,
wobei als Substituenten infrage kommen: Fluor, Chlor, Brom, Cyano, Nitro, Methyl, Ethyl, n- oder i-Propyl, n-, i-, s- oder t-Butyl, Methoxy, Ethoxy, n- oder i-Propoxy, Methylthio, Difluormethyl, Trifluormethyl, Trifluormethoxy, Trifluormethylthio, Methoxycarbonyl, Ethoxycarbonyl, Methoximinomethyl, Ethoximinomethyl, Methoximinoethyl, Ethoximinoethyl oder gegebenenfalls ein- bis dreifach, gleich oder verschieden durch Fluor, Chlor, Brom, Methyl und/ oder Ethyl substituiertes Phenyl,

R für Methyl oder Ethyl steht und

X für Schwefel steht,

ausgenommen 5-(2-Chlorphenyl)- und 5-(3-Nitrophenyl)-2-methylsulfonyl-1,3,4-oxadiazol.

Ganz besonders hervorzuheben sind Verbindungen der Formel (I), in welcher

Ar für 4-Chlorphenyl oder 4-Methylphenyl steht,

R für Methyl steht und
X für Sauerstoff steht oder
Ar für 4-Chlorphenyl steht,
R für Methyl und
X für Schwefel steht.

Im einzelnen seien außer den bei der Herstellungsbeispielen genannten Verbindungen die folgenden heterocyclisch substituierten Sulfone der allgemeinen Formel (I) genannt:

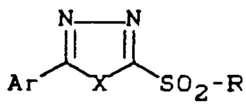

(I)

| Ar | R | X | Ar | R | X |
|---|---|---|---|---|---|
| 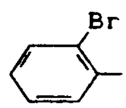 | $CH_3$ | O/S | 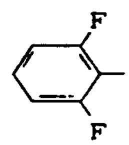 | $CH_3$ | O/S |
| 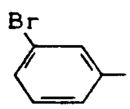 | $CH_3$ | O/S | 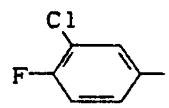 | $CH_3$ | O/S |
| 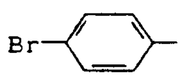 | $CH_3$ | O/S | 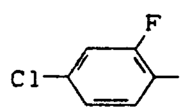 | $CH_3$ | O/S |
| 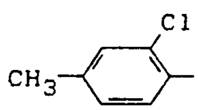 | $CH_3$ | O/S | 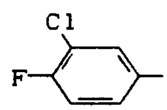 | $CH_3$ | O/S |
| 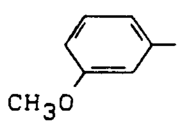 | $CH_3$ | O/S | 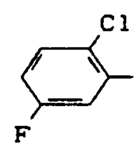 | $CH_3$ | O/S |
| 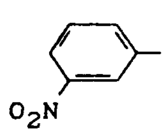 | $CH_3$ | O/S | 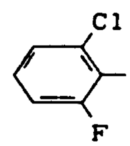 | $CH_3$ | O/S |

| Ar | R | X | Ar | R | X |
|---|---|---|---|---|---|
| 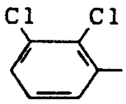 | $CH_3$ | O/S | 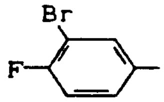 | $CH_3$ | O/S |
| 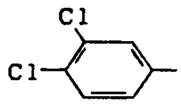 | $CH_3$ | O/S | 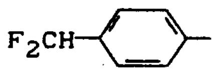 | $CH_3$ | O/S |
| 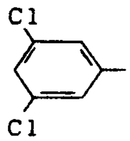 | $CH_3$ | O/S | 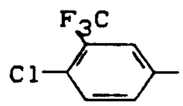 | $CH_3$ | O/S |
| 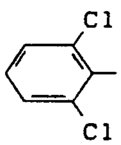 | $CH_3$ | O/S | 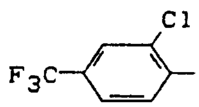 | $CH_3$ | O/S |
| 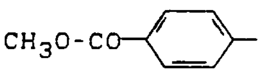 | $CH_3$ | O/S | 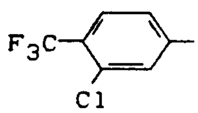 | $CH_3$ | O/S |
| 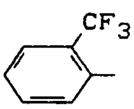 | $CH_3$ | O/S | 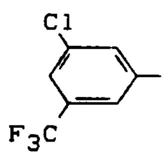 | $CH_3$ | O/S |
| 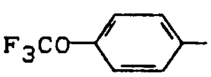 | $CH_3$ | O/S | 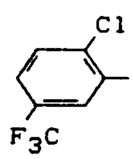 | $CH_3$ | O/S |
| 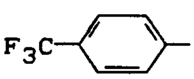 | $CH_3$ | O/S | 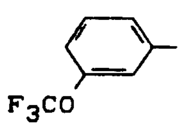 | $CH_3$ | O/S |

| Ar | R | X | Ar | R | X |
|---|---|---|---|---|---|
| 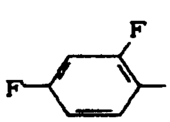 | $CH_3$ | O/S | 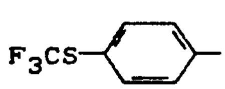 | $CH_3$ | O/S |

Die erfindungsgemäß verwendbaren heterocyclisch substituierten Sulfone der Formel (I) sind bekannt (vergleiche z.B. J. Chem. Soc., Perkin Trans. 1, 1983, 967-971; J. Am. Chem. Soc. 105, 902-906 [1983]; JP 52010421/1977; Farmaco, Ed. Sci., 32, 414-429 [1977] bzw. CA 87: 95447j; Agric. Biol. Chem. 40, 17-21

[1976] bzw. CA 84: 121736n; Indian J. Chem.. 7, 760-765 [1969]; Bull. Chim. Farm. 106, 826-836 [1967] bzw. CA 69: 27340x) oder erhältlich mit Hilfe allgemein üblicher, bekannter Verfahren (vergleiche z.B. J. Amer. Chem. Soc. 105, 902 [1983]; J. Amer. Chem. Soc. 77, 400 [1955] sowie die Herstellungsbeispiele).

Die erfindungsgemäß verwendbaren Wirkstoffe weisen eine starke Wirkung gegen phytopathogene Pilze auf und können zur Bekämpfung von unerwünschten Schadorganismen in der Landwirtschaft praktisch eingesetzt werden. Die Wirkstoffe sind für den Gebrauch als Pflansenschutzmittel insbesondere als Fungizide geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Beispielhaft aber nicht begrenzend seien einige Erreger von pilzlichen Erkrankungen, die unter die oben aufgezählten Oberbegriffe fallen, genannt:
Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder Pseudoperonospora cubensis;
Plasmopara-Arten, wie beispielsweise Plasmopara viticola;
Peronospora-Arten, wie beispielsweise Peronospora pisi oder P. brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielsweise Pyrenophora teres oder P. graminea
(Konidienform: Drechslera, Syn: Helminthosporium);
Cochliobolus-Arten, wie beispielsweise Cochliobolus sativus
(Konidienform: Drechslera, Syn: Helminthosporium);
Uromyces-Arten, wie beispielsweise Uromyces appendiculatus;
Puccinia-Arten, wie beispielsweise Puccinia recondita;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielsweise Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Dabei lassen sich die erfindungsgemäß verwendbaren Verbindungen mit besonders gutem Erfolg zur Bekämpfung von Getreidekrankheiten, wie beispielsweise gegen den Erreger des echten Getreidemehltaus (Erysiphe graminis) oder gegen den Erreger der Braunspelzigkeit an Weizen (Septoria nodorum) oder gegen den Erreger der Netzfleckenkrankheit der Gerste (Pyrenophora teres), gegen Fusarium-Arten an Getreide oder zur Bekämpfung von Krankheiten im Reisanbau, wie beispielsweise gegen den Erreger der Reisfleckenkrankheit (Pyricularia oryzae) oder zur Bekämpfung von Krankheiten im Obst- und Gemüseanbau, wie beispielsweise gegen den Erreger des Apfelschorfes (Venturia inaequalis) einsetzen. Dabei zeigen die erfindungsgemäß verwendbaren Wirkstoffe neben protektiven Eigenschaften auch kurative und systemische Wirksamkeit.

Die Wirkstoffe können in Abhängigkeit von ihren jeweiligen physikalischen und/oder chemischen Eigenschaften in übliche Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als

Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. AlkylarylpolyglykolEther, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen in Mischung mit anderen bekannten Wirkstoffen vorliegen wie Fungizide, Insektizide, Akarizide und Herbizide sowie in Mischungen mit Düngemitteln und Wachstumsregulatoren.

Die Wirkstoffe konnen als solche, in Form ihrer Formulierungen oder den daraus bereiteten Anwendungsformen wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verstäuben, Verschäumen, Bestreichen usw.. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g benötigt.

Bei Behandlung des Bodens sind Wirkstoffkonzentrationen von 0,00001 bis 0,1 Gew.-%, vorzugsweise von 0,0001 bis 0,02 % am Wirkungsort erforderlich.

Herstellungsbeispiele:

Beispiel 1:

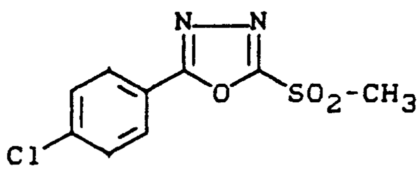

Zu 123,5 g (0,546 Mol) 5-(4-Chlorphenyl)-2-methylthio-1,3,4-oxadiazol in 1000 ml Eisessig gibt man bei Raumtemperatur tropfenweise unter Rühren eine Lösung von 180,9 g (1,145 Mol) Kaliumpermanganat in 3600 ml Wasser. Anschließend gibt man 40prozentige wässrige Natriumhydrogensulfitlösung bis zur Entfärbung zu, filtriert ausgefallenes Reaktionsprodukt ab und kristallisiert aus Ethanol um.

Man erhält 92,5 g (66 % der Theorie) an 5-(4-Chlorphenyl)-2-methylsulfonyl-1,3,4-oxadiazol vom Schmelzpunkt 159 ° C bis 160 ° C.

Herstellung der Ausgangsverbindung:

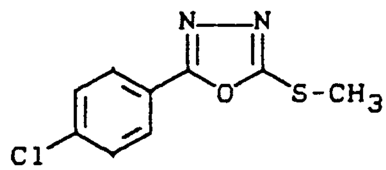

Zu einer Suspension von 34,1 g (0,2 Mol) p-Chlorbenzoylhydrazid in 300 ml Ethanol gibt man 12 g (0,21 Mol) Kaliumhydroxid in 18 ml Wasser gelöst zu und tropft anschließend langsam bei Raumtemperatur unter Rühren 16,5 g (0,22 Mol) Schwefelkohlenstoff zu. Nach beendeter Zugabe verdünnt man die Mischung mit 200 ml Ethanol, erhitzt für 16 Stunden auf Rückflußtemperatur, gibt dann tropfenweise unter Rühren 30,8 g (0,22 Mol) Methyliodid zu, kühlt anschließend auf 0 ° C ab, rührt 30 Minuten bei dieser Temperatur, saugt ausgefallenes Reaktionsprodukt ab und kristallisiert aus Ethanol um.

Man erhält 38 g (84 % der Theorie) an 5-(4-Chlorphenyl)-2-methylthio-1,3,4-oxadiazol vom Schmelzpunkt 121 ° C bis 123 ° C.

In entsprechender Weise erhält man die folgenden heterocyclisch substituierten Sulfone der allgemeinen Formel (I)

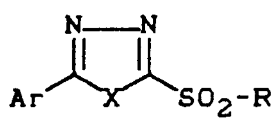

(I)

| Bsp.Nr. | Ar | R | X | Schmelzpunkt °C |
|---|---|---|---|---|
| 2 | 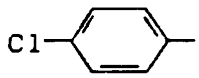 | $CH_3$ | S | 190-192 |
| 3 | 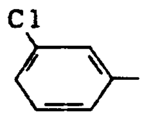 | $CH_3$ | O | 128-130 |
| 4 | 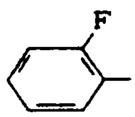 | $CH_3$ | O | 105 |
| 5 | 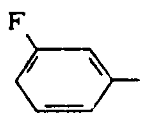 | $CH_3$ | O | 140 |
| 6 | 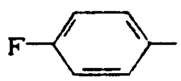 | $CH_3$ | O | 123 |
| 7 | 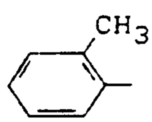 | $CH_3$ | O | 111-113 |
| 8 | 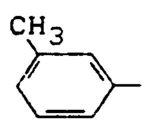 | $CH_3$ | O | 103 |
| 9 | 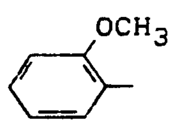 | $CH_3$ | O | 146-148 |
| 10 | 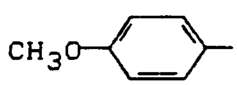 | $CH_3$ | O | 154-156 |

| Bsp.Nr. | Ar | R | X | Schmelzpunkt °C |
|---|---|---|---|---|
| 11 | 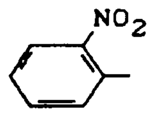 | $CH_3$ | O | 108-110 |
| 12 | 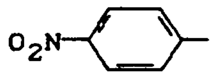 | $CH_3$ | O | 168-172 |
| 13 | 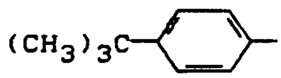 | $CH_3$ | O | 94-96 |
| 14 | 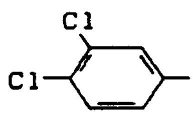 | $CH_3$ | O | 166-168 |
| 15 | 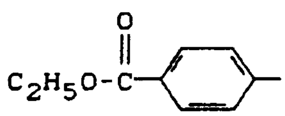 | $CH_3$ | O | Öl |
| 16 | 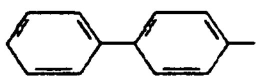 | $CH_3$ | O | 170-173 |
| 17 | 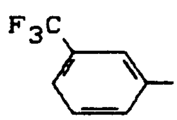 | $CH_3$ | O | 90-94 |
| 18 | 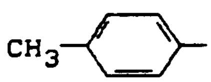 | $CH_3$ | O | Öl |
| 19 | 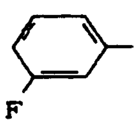 | $CH_3$ | S | 91 |
| 20 | 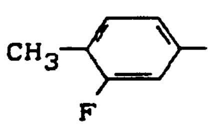 | $CH_3$ | S | 112 |
| 21 | 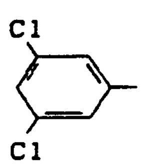 | $CH_3$ | S | 138 |

| Bsp.Nr. | Ar | R | X | Schmelzpunkt °C |
|---|---|---|---|---|
| 22 | 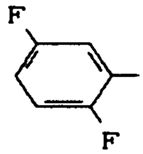 | $CH_3$ | S | 124 |
| 23 | 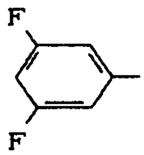 | $CH_3$ | S | 125 |
| 24 | 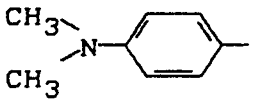 | $CH_3$ | S | |
| 25 | 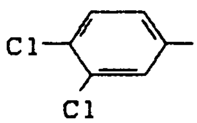 | $CH_3$ | S | 181 |
| 26 | 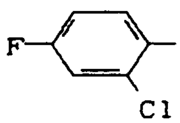 | $CH_3$ | S | 91 |
| 27 | 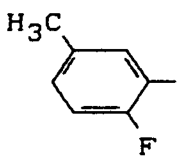 | $CH_3$ | S | 84 |
| 28 | 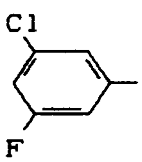 | $CH_3$ | S | 107 |

| Bsp.Nr. | Ar | R | X | Schmelzpunkt °C |
|---|---|---|---|---|
| 29 | 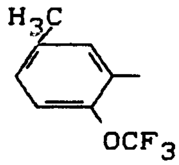 | $CH_3$ | S | 58 |
| 30 | 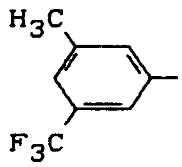 | $CH_3$ | S | 82 |

Anwendungsbeispiele:

In den folgenden Anwendungsbeispielen wurden die nachstehend aufgeführten Verbindungen als Vergleichssubstanzen eingesetzt:

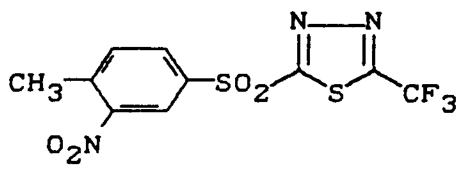

(A)

2-[(4-Methyl-3-nitrophenyl)-sulfonyl]-5-trifluormethyl-1,3,4-thiadiazol (bekannt aus DE-OS 25 33 604) oder

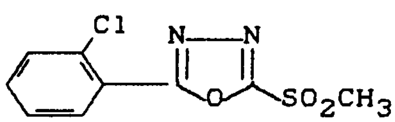

(B)

5-(2-Chlorphenyl)-2-methylsulfonyl-1,3,4-oxadiazol (bekannt aus Agr. Biol. Chem. 40 (1), pg. 17-21 (1976)).

Beispiel A

Botrytis-Test (Buschbohne) / protektiv

Lösungsmittel: 4,7 Gewichtsteile Aceton

Emulgator: 0,3 Gewichtsteile Alkyl-aryl-polyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden auf jedes Blatt 2 kleine mit Botrytis cinerea bewachsene Agarstückchen aufgelegt. Die inokulierten Pflanzen werden in einer abgedunkelten, feuchten Kammer bei 20°C aufgestellt. 3 Tage nach der Inokulation wird die Größe der Befallsflecken auf den Blättern ausgewertet.

Eine deutliche Überlegenheit in der Wirksamkeit gegenüber dem Stand der Technik zeigen in diesem Test z.B. die Verbindungen gemäß folgender Herstellungsbeispiele:

Tabelle A

Botrytis - Test (Bohne) / protektiv

| Wirkstoff | Wirkungsgrad in % der unbehandelten Kontrolle bei einer Wirkstoffkonzentration von 100 ppm |
|---|---|
| Bekannt: | |
| 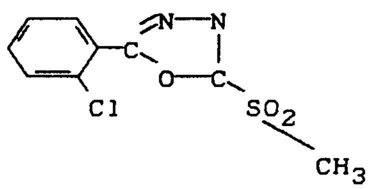 | 0 |
| Erfindungsgemäß: | |
| 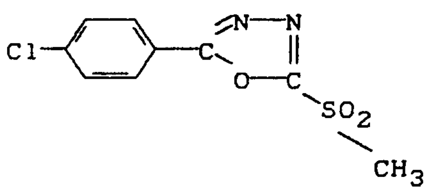 | 100 |
| 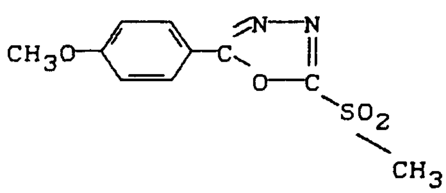 | 52 |
| 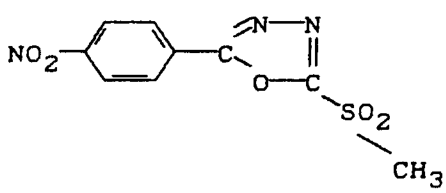 | 60 |

Tabelle A

Botrytis - Test (Bohne) / protektiv

| Wirkstoff | Wirkungsgrad in % der unbehandelten Kontrolle bei einer Wirkstoffkonzentration von 100 ppm |
|---|---|
| 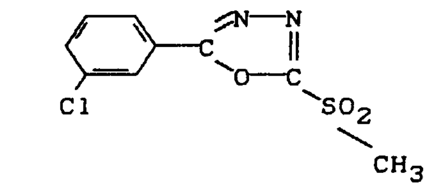 | 99 |
| 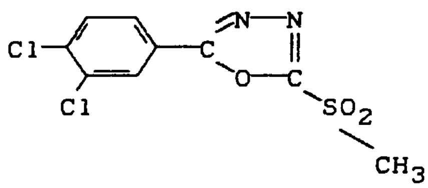 | 100 |
| 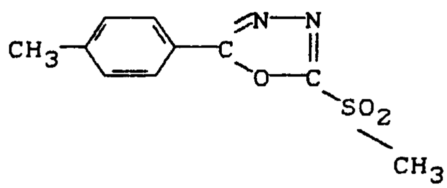 | 55 |
| 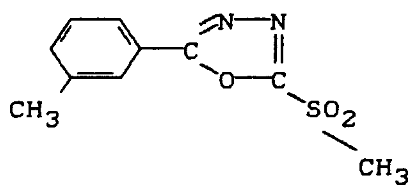 | 74 |

Beispiel B

Venturia-Test (Apfel) / protektiv

Lösungsmittel: 4,7 Gewichtsteile Aceton

Emulgator: 0,3 Gewichtsteile Alkyl-aryl-polyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wäßrigen Konidiensuspension des Apfelschorferregers (Venturia inaequalis) inokuliert und verbleiben dann 1 Tag bei 20 °C und 100 % relativer Luftfeuchtigkeit in einer Inkubationskabine.

Die Pflanzen werden dann im Gewächshaus bei 20 °C und einer relativen Luftfeuchtigkeit von ca. 70 % aufgestellt.

12 Tage nach der Inokulation erfolgt die Auswertung.

Eine deutliche Überlegenheit in der Wirksamkeit gegenüber dem Stand der Technik zeigt in diesem Test z.B. die Verbindung gemäß Herstellungsbeispiel 1.

Tabelle B

Venturia-Test (Apfel) / protektiv

| Wirstoff | Wirkungsgrad in % der unbehandelten Kontrolle bei einer Wirkstoffkonzentration von 5 ppm |
| --- | --- |
| 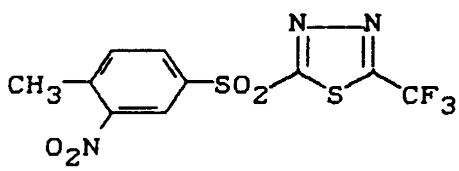 (A) (bekannt) | 73 |
| 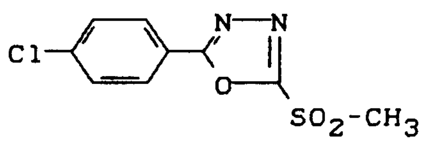 (1) | 93 |

**Patentansprüche**

**1.** Verwendung von heterocyclisch substituierten Sulfonen der allgemeinen Formel (I),

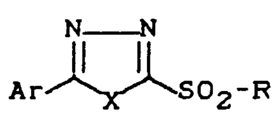

(I)

in welcher

Ar für einfach bis dreifach, gleich oder verschieden substituiertes Phenyl steht, wobei als Substituenten infrage kommen: Halogen, Cyano, Nitro, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen, jeweils geradkettiges oder verzweigtes Alkoxycarbonyl oder Alkoximinoalkyl mit jeweils 1 bis 4 Kohlenstoffatomen in den einzelnen Alkylteilen oder gegebenenfalls einfach bis fünffach, gleich oder verschieden durch Halogen und/oder geradkettiges oder verzweigtes Alkyl mit 1 bis 4 Kohlenstoffatomen substituiertes Phenyl,

R für geradkettiges oder verzweigtes Alkyl mit 1 bis 4 Kohlenstoffatomen steht und

X für Sauerstoff oder Schwefel steht, ausgenommen die Verbindungen 5-(2-Chlorphenyl)- und 5-(3-Nitrophenyl)-2-methylsulfonyl-1,3,4-oxadiazol,

zur Bekämpfung von phytopathogenen Pilzen in der Landwirtschaft.

**3.** Verwendung von Verbindungen gemäß Anspruch 1, in welcher

Ar für ein- bis dreifach, gleich oder verschieden substituiertes Phenyl steht, wobei als Substituenten infrage kommen: Fluor, Chlor, Brom, Cyano, Nitro, Methyl, Ethyl, n-oder i-Propyl, n-, 1-, s- oder t-Butyl, Methoxy, Ethoxy, n- oder i-Propoxy, Methylthio, Difluormethyl, Trifluormethyl, Trifluormethoxy, Trifluormethylthio, Methoxycarbonyl, Ethoxycarbonyl, Methoximinomethyl, Ethoximinomethyl, Methoximinoethyl, Ethoximinoethyl oder gegebenenfalls ein- bis dreifach, gleich oder verschieden durch Fluor, Chlor, Brom, Methyl und/ oder Ethyl substituiertes Phenyl,

R für Methyl oder Ethyl steht und

X für Sauerstoff steht,

ausgenommen 5-(2-Chlorphenyl)- und 5-(3-Nitrophenyl)-2-methylsulfonyl-1,3,4-oxadiazol,

zur Bekämpfung von phytopathogenen Pilzen in der Landwirtschaft.

**3.** Verwendung von Verbindungen gemäß Anspruch 1 der Formel (I), in welcher

Ar für ein- bis dreifach, gleich oder verschieden substituiertes Phenyl steht, wobei als Substituenten infrage kommen: Fluor, Chlor, Brom, Cyano, Nitro, Methyl, Ethyl, n-oder i-Propyl, n-, i-, s- oder t-Butyl, Methoxy, Ethoxy, n- oder i-Propoxy, Methylthio, Difluormethyl, Trifluormethyl, Trifluormethoxy, Trifluormethylthio, Methoxycarbonyl, Ethoxycarbonyl, Methoximinomethyl, Ethoximinomethyl, Methoximinoethyl, Ethoximinoethyl oder gegebenenfalls ein- bis dreifach, gleich oder verschieden durch Fluor, Chlor, Brom, Methyl und/ oder Ethyl substituiertes Phenyl,

R für Methyl oder Ethyl steht und

X für Schwefel steht,

zur Bekämpfung von phythopathogenen Pilzen in der Landwirtschaft.

**4.** Verwendung von 5-(4-Chlorphenyl)-2-methylsulfonyl-1,3,4-oxadiazol gemäß Anspruch 1 zur Bekämpfung von phytopathogenen Pilzen in der Landwirtschaft.

**5.** Verwendung von 5-(4-Methylphenyl)-2-methylsulfonyl-1,3,4-oxadiazol gemäß Anspruch 1 zur Bekämpfung von phytopathogenen Pilzen in der Landwirtschaft.

**6.** Verwendung von 5-(4-Chlorphenyl)-2-methylsulfonyl-1,3,4-thiadiazol gemäß Anspruch 1 zur Bekämpfung von phytopathogenen Pilzen in der Landwirtschaft.

**7.** Schädlingsbekämpfungsmittel, gekennzeichnet durch einen Gehalt an mindestens einem heterocyclisch substituierten Sulfon der Formel (I) nach den Ansprüchen 1 bis 4.

**8.** Verfahren zur Bekämpfung von Schädlingen, dadurch gekennzeichnet, daß man mindestens ein heterocyclisch substituiertes Sulfon der Formel (I) nach den Ansprüchen 1 bis 6 auf Schädlinge und/oder ihren Lebensraum einwirken läßt.

**9.** Verfahren zur Herstellung von Schädlingsbekämpfungsmitteln, dadurch gekennzeichnet, daß man heterocyclisch substituiertes Sulfon der Formel (I) nach den Ansprüchen 1 bis 6 mit Streckmitteln und/oder oberflächenaktiven Mitteln vermischt.

Europäisches Patentamt

# EUROPÄISCHER RECHERCHENBERICHT

Nummer der Anmeldung

EP 91 11 7032

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X,D | AGRICULTURAL AND BIOLOGICAL CHEMISTRY, Band 40, Nr. 1, 1976, Seiten 17-21, Tokyo, JP; S. GIRI et al.: "Studies in oxadiazoles synthesis of some 2-mercapto-1,3,4-oxadiazoles and related compounds as potential fungicides" * Seite 17, rechte Spalte, Absatz 1; Tabel III * --- | 1-9 | A 01 N 43/82<br>C 07 D 271/10<br>C 07 D 285/12 |
| X | CHEMICAL ABSTRACTS, Band 108, Nr. 17, 25. April 1988, Seite 738, Zusammenfassung Nr. 150375p, Columbus, Ohio, US; S.A. ABDUL-HAFIDH et al.: "Synthesis and antimicrobial evaluation of new derivatives of 5-phenyl-1,3,4-thiadiazoline-2-thione", & J. BIOL. SCI. RES. 1987, 18(2), 131-40 * Zusammenfassung * ----- | 1-9 | |
| | | | RECHERCHIERTE SACHGEBIETE (Int. Cl.5) |
| | | | A 01 N |

Der vorliegende Recherchenbericht wurde für alle Patentansprüche erstellt

| Recherchenort | Abschlußdatum der Recherche | Prüfer |
|---|---|---|
| DEN HAAG | 24-01-1992 | DECORTE D. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)